# EUROPÄISCHE PATENTSCHRIFT

(11) **EP 2 537 479 B1**
(45) Veröffentlichungstag und Bekanntmachung des Hinweises auf die Patenterteilung: **16.04.2014**
(21) Anmeldenummer: 11170577.8
(22) Anmeldetag: 20.06.2011
(51) Int. Cl.: A61B 18/12, A61B 18/16, A61B 18/00

(54) **Steuerung eines medizinischen Geräts in Abhängigkeit von der Neutralektrodenimpedanz**
Controlling a medical device depending on neutral electrode impedance
Commande d'un appareil médical en fonction de l'impédance des électrodes neutres

(43) Veröffentlichungstag der Anmeldung: 26.12.2012
(73) Patentinhaber: ERBE Elektromedizin GmbH, 72072 Tübingen (DE)
(72) Erfinder: Hagg, Martin, 72827 Wannweil (DE); Selig, Peter, 72379 Hechingen (DE); Beller, Jürgen, 72810 Gomaringen (DE)
(74) Vertreter: Rüger, Barthelt & Abel

(56) Entgegenhaltungen:
- WO-A1-2009/109197
- DE-A1- 3 239 640
- DE-T2- 68 924 918

## Beschreibung

Die Erfindung betrifft ein Verfahren zur Steuerung eines medizinischen Geräts in Abhängigkeit von der an der Neutralelektrode erfassten Impedanz sowie eine entsprechende elektrochirurgische Einrichtung.

Bei elektrochirurgischen Geräten, insbesondere bei der monopolaren HF-Chirurgie, dient ein elektromedizinisches Gerät zur Speisung eines vom Chirurgen zu führenden Instruments, mit dem der Chirurg gewünschte Operationen, bspw. Gewebefusionen, Koagulationen, Abtragungen oder dergleichen, vornimmt. Ist das Instrument monopolar, wird es über eine Leitung mit HF-Leistung versorgt. Zum Schließen des Stromkreises dient eine Neutralelektrode, die großflächig an dem Patienten zu befestigen ist. Damit wird ein Strompfad von dem aktiven Instrument über den Körper des Patienten zurück zu dem elektromedizinischen Gerät bereitgesellt. Über die Neutralelektrode soll der Strom möglichst gewebeschonend geleitet werden. Temperaturerhöhungen sollen an der Neutralelektrode gering bleiben, um an dieser Stelle keine thermischen Gewebeeffekte oder gar Schädigungen zu erzeugen. Deshalb gilt es, die Stromdichte zu begrenzen. Dazu wird die Neutralelektrode großflächig ausgeführt. Je größer die Kontaktfläche der Neutralelektrode ist, desto geringer ist die sich ergebende Stromdichte und die dadurch hervorgerufen Erwärmung.

Bei großflächigen Elektroden muss auf ihre möglichst vollständige Anlage an dem Patienten geachtet werden, um lokale Stromkonzentrationen zu vermeiden. Deshalb wird die Elektrode üblicherweise in zwei Abschnitte aufgeteilt, die beide an der Haut des Patienten anliegen. Die zwischen beiden Abschnitten zu messende Impedanz beziehungsweise der Übergangswiderstand wird mit einem Grenzwert verglichen. Überschreitet die Impedanz den Grenzwert, wird eine Aktivierung des HF-chirurgischen Geräts unterbunden.

Eine derartige Überwachungsanordnung wird beispielsweise in der DE 32 39 640 A1 beschrieben. Hier kann die obere Grenze eines Sollbereichs der Impedanz an den Patienten angepasst werden. Auch die Überwachungseinrichtung der DE 689 24 918 T2 beinhaltet eine Stelleinrichtung zum manuellen Einstellen eines Widerstandsschwellenwerts innerhalb eines festen Widerstandsbereichs. Die Vorrichtung für die Thermochirurgie der WO 2009/109197 A1 mit einer Anwendungselektrode und mehreren Gegenelektroden weist Impedanzmessmittel auf, die die Impedanzmessung in mehreren Messkreisen gestatten, wobei jeder Messkreis über mindestens eine Gegenelektrode führt.

Der Grenzwert für die Gewebeimpedanz muss relativ niedrig eingestellt werden, um eine fehlerhaft, d.h. unvollständige Anlage der Neutralelektrode wirklich erfassen zu können. Allerdings führt dies bei Patienten mit trockener Haut oder einer dicken Schicht subkutanen Fettgewebes zum Überschreiten der Impedanzschwelle und somit zur Deaktivierung des HF-chirurgischen Geräts, obwohl die Neutralelektrode ordnungsgemäß an dem Patienten befestigt ist. Dies führt im OP-Saal durchaus zu Schwierigkeiten. Die geübte Praxis, durch Reinigen oder Anfeuchten der Haut, Verwendung größerer Neutralelektroden oder Suche nach geeigneteren Anbringstellen, den Grenzwert zu unterschreiten, führt mindestens zu einer Störung des gewohnten Operationsablaufs und auch zu zusätzlichem Zeitaufwand. Eine Erhöhung des Schwellwerts ist wegen der damit einhergehenden Gefahr, bei Patienten mit niedrigem Hautwiderstand eine fehlerhafte Anlage der Elektrode nicht zu erkennen, keine wirkliche Alternative. Es können dann nämlich an der betreffenden Anbringstelle Hautschädigungen auftreten.

Davon ausgehend ist es Aufgabe der Erfindung, ein Verfahren zur Steuerung eines elektromedizinischen Geräts anzugeben, das einerseits eine hohe Patientensicherheit erbringt und andererseits den Betriebsablauf bei Operationen möglichst frei von größeren Störungen hält.

Diese Aufgabe wird mit dem Verfahren nach Anspruch 1 gelöst. Außerdem wird die Aufgabe durch ein elektrochirurgisches Gerät nach Anspruch 10 gelöst:
Nach dem erfindungsgemäßen Konzept wird das korrekte Anlegen der Neutralelektrode durch eine Impedanzmessung zwischen wenigstens zwei Abschnitte der Neutralelektrode erfasst. Die erfasste Impedanz kennzeichnet den Übergangswiderstand von der Neutralelektrode zu dem Patienten. Liegt der Wert der erfassten Impedanz unter einem ersten Grenzwert, wird der Betrieb des Geräts uneingeschränkt freigegeben. Liegt die elektrische Impedanz zwischen den zwei Abschnitten jedoch über diesem ersten Grenzwert, wird der Betrieb des elektromedizinischen Geräts zunächst gesperrt. Dazu wird ein entsprechendes Warnsignal generiert, das optischer und/oder akustischer Natur sein kann. Beispielsweise kann es sich um ein Tonsignal, verbunden mit einer Bild- oder Textausgabe auf einem Bildschirm handeln. Auf das Warnsignal hin kann der Bediener die korrekte vollflächige Anlage der Neutralelektrode überprüfen und nachfolgend einen entsprechenden Nutzerbefehl eingeben, der die korrekte Anlage der Neutralelektrode bestätigt. Bei dem Nutzerbefehl kann es sich um einen Tastendruck oder um sonstige Eingaben handeln, mit denen die korrekte Anlage der Elektrode bestätigt wird.

Durch die Eingabe dieses Nutzerbefehls kann die Sperrung aufgehoben werden, wodurch der Betrieb des elektrochirurgischen Geräts möglich wird, obwohl die Impedanz an der Neutralelektrode den ersten Grenzwert überschritten hat. Zur Aufhebung der Sperrung kann der Grenzwert zumindest temporär auf einen höheren Wert hin verschoben werden.

Eine Schädigung der Haut des Patienten ist nach manueller Nachkontrolle des Sitzes der Elektrode und nachfolgender Aufhebung der Sperrung durch eine Nutzereingabe nicht zu erwarten, weil die erhöhte Impedanz nicht auf fehlerhafter Anlage der Neutralelektroden, sondern auch sonstige Einflüsse, wie z.B. dickes Unterhautfettgewebe, zurück geht. Somit weist die Erfindung einen Weg trotz Überwachung der Anlage der Neutralelektrode durch Vergleich der Impedanz mit einem Schwellwert, die Störung des chirurgischen Betriebs zu minimieren.

Erfindungsgemäß kann die Aufhebung der Sperrung bei Überschreiten des ersten Grenzwerts nur vorgenommen werden, wenn die erfasste Impedanz niedriger ist als ein zweiter höherer Grenzwert. Wird auch der zweite höhere Grenzwert überschritten, ist eine Aufhebung der Sperrung durch den Bediener nicht möglich. Dieses Verhalten kann z.B. erreicht werden, indem der erste Schwellwert zumindest temporär durch den zweiten, höheren Schwellwert ersetzt wird.

Alternativ kann vorgesehen werden, dass die Aufhebung der Sperrung bei Überschreiten des zweiten Grenzwerts nur in Ausnahmefällen, z.B. nach erneuter und gegebenenfalls deutlicher Warnung möglich ist. Es kann auch vorgesehen werden, dass das Gerät dann nur für kurze Zeit betrieben werden kann, d.h. dass die Betriebszeit auf ein oder mehrere kurze Intervalle beschränkt ist. Zusätzlich oder alternativ kann vorgesehen sein, dass das Gerät, insbesondere sein Generator, in diesem Falle nur mit reduzierter Leistung betreibbar ist.

Bei der bevorzugten Ausführungsform unterscheidet die Steuereinrichtung des elektromedizinischen Geräts drei Zustände: A) einen Zustand mit Impedanz unterhalb des ersten Grenzwerts. Hier ist der Betrieb des elektromedizinischen Geräts uneingeschränkt möglich. B) eine Impedanz, die zwischen dem ersten und dem zweiten Grenzwert liegt. Hier ist ein Betrieb des elektromedizinischen Geräts nur eingeschränkt nach manueller Aufhebung der zunächst automatisch eingetretenen Sperrung des Geräts möglich. C) liegt die Impedanz oberhalb des zweiten Grenzwerts, wird der weitere Betrieb des medizinischen Geräts vorzugsweise ganz gesperrt.

Die genannte Funktion ermöglicht es, auch bei Patienten mit kritischen Gewebeimpedanzen nahezu ohne Zeitverlust und zusätzlichen Aufwand mit dem chirurgischen Eingriff beginnen zu können. Trotzdem wird dadurch die Sicherheit in der Anwendung für Patienten mit geringen Gewebeimpedanzen nicht eingeschränkt und eine automatische Überwachung der Qualität der Anlage der Neutralelektrode weiterhin sichergestellt. Die Impedanz kann indes während des Betriebs des elektrochirurgischen Geräts wiederholt oder fortwährend überwacht werden. Die wiederholte Impedanzüberprüfung kann z.B. periodisch oder auch von Zeit zu Zeit erfolgen. Wird beispielsweise bei Überschreiten des ersten Grenzwerts und nachfolgender manueller Aufhebung der Sperrung des Betriebs die Impedanz fortwährend oder wiederholt überwacht, kann überprüft werden, ob die Impedanz nach und nach etwas abfällt. Dies kann durch das Erwärmen der Neutralelektrode auf die Oberflächentemperatur des Körpers und gegebenenfalls Transpiration unter der Elektrode geschehen. Von der Haut können Elektrolyte abgegeben werden, die die Leitfähigkeit erhöhen und die Impedanz vermindern.

Wenn ein Unterschreiten des ersten Grenzwerts festgestellt wird, kann dieser gewissermaßen reaktiviert werden und hinfort wieder als Grenzwert gelten. Sollte der erste Grenzwert wieder überschritten werden, kann in diesem Fall wiederum eine Warnung ausgegeben und/oder der Betrieb des elektromedizinischen Geräts erneut gesperrt werden. Der erste Grenzwert kann hier etwas verändert, bspw. zusätzlich vermindert werden, um eine Hysterese für das Hin- und Herschalten zwischen dem ersten und dem zweiten Grenzwert zu bewirken. Es ist auch möglich, dass das Gerät im Falle einer erneuten Überschreitung des ersten Grenzwerts oder, wenn es manuell entsperrt worden ist, bei Überschreitung des zweiten Grenzwerts zum Anlegen einer größeren Neutralelektrode auffordert. Auch dies kann dazu dienen, Zeitverluste im OP-Saal zu vermeiden.

Weiter ist es möglich, anstelle einer vollständigen Sperrung bei Überschreiten des ersten Grenzwerts und Unterschreiten des zweiten Grenzwerts einen Betrieb mit reduzierter Leistung zuzulassen. Somit wird der Betrieb von chirurgischen Instrumenten, die mit wenig Leistung betrieben werden, weiter ermöglicht, obwohl an der Neutralelektrode eine größere Impedanz erfasst wird. Beispielsweise kann das Gerät in diesem Fall bei einer Impedanz unter dem ersten Grenzwert einen Betrieb mit uneingeschränkt hoher Leistung zulassen. Weiter kann das Gerät bei einer Impedanz über dem ersten, jedoch unter dem zweiten Grenzwert einen Betrieb mit verminderter Leistung zulassen. Bei Überschreiten des zweiten Grenzwerts kann vorgesehen sein, dass das Gerät keinen Betrieb mehr zulässt. Optional kann im zweiten Fall, wenn die Impedanz zwischen dem ersten und dem zweiten Grenzwert liegt, auch eine manuelle Entsperrung vorgenommen werden, wonach der Betrieb mit uneingeschränkter Leistung möglich ist.

An dem genannten Verfahren sind zahlreiche Abwandlungen möglich. Dazu wird auf die Zeichnung und nachfolgende Beschreibung einer beispielhaften Ausführungsform verweisen. Es zeigen:
Figur 1 eine elektrochirurgisches Gerät mit angeschlossenem Instrument und Neutralelektrode,
Figur 2 die Neutralelektrode nach Figur 1,
Figur 3 das elektrochirurgische Gerät als Blockschaltbild,
Figur 4 einen beispielhaften zeitlichen Verlauf von Gewebeimpedanzmessungen und zugehörig festgelegten Grenzwerten und
Figur 5 ein Flussbild für eine beispielhafte Verwirklichung des erfindungsgemäßen Verfahrens.

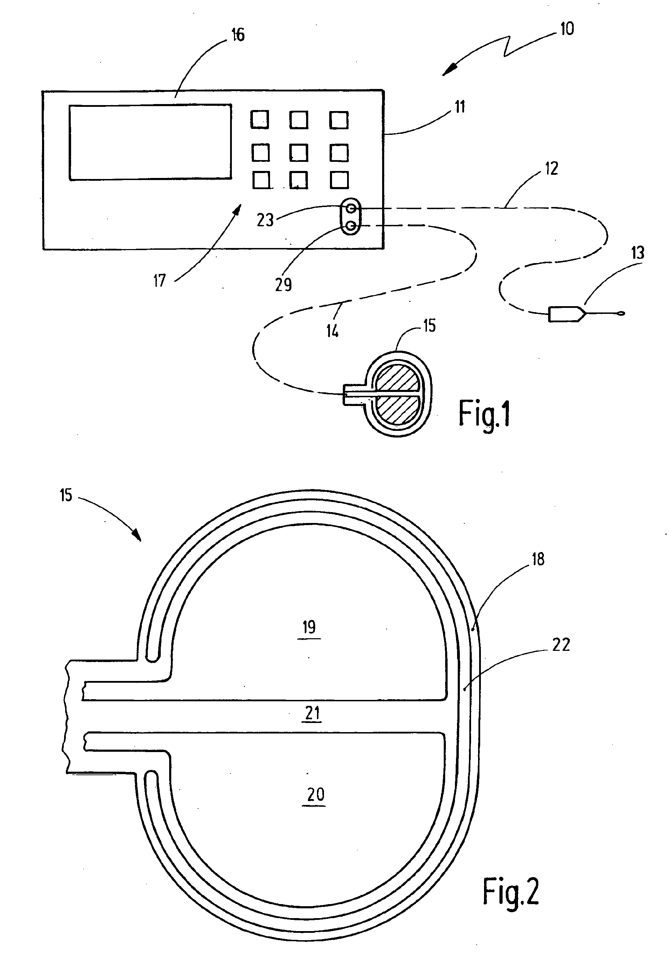

In Figur 1 ist eine elektrochirurgische Einrichtung 10 veranschaulicht, zu der ein elektromedizinisches Gerät 11 gehört, das über eine Leitung 12 ein chirurgisches Instrument 13 speist und über eine Leitung 14 mit einer Neutralelektrode 15 verbunden ist. Während das chirurgische Instrument 13 in der Hand des Chirurgen dazu dient, an einem Patienten chirurgische Eingriffe vorzunehmen, ist die Neutralelektrode 15 dazu vorgesehen den Stromkreis über den Patienten zu schließen. Sie wird großflächig an einer geeigneten Stelle des Patienten, beispielsweise seinem Oberschenkel befestigt.

Das zur Speisung des Instruments 13 dienende Gerät 11 weist mindestens eine Anzeigeeinrichtung 16, bspw. in Gestalt eines Bildschirms, und Bedienelemente 17, zum Beispiel in Form von Tasten, Bedienknöpfen oder dergleichen, auf. Weitere oder andere Bedienelemente und Anzeigeeinrichtungen können vorgesehen sein.

Die Neutralelektrode 15 ist in Figur 2 gesondert veranschaulicht. Sie besteht aus einem elektrisch nicht leitenden Träger 18, auf dem mindestens zwei großflächige metallische Abschnitte 19, 20 angeordnet sind. Diese können als dünne Metallfolien oder beispielsweise als metallische Beschichtungen ausgebildet sein. Sie sind durch einen Abstand 21 voneinander getrennt und somit gegeneinander elektrisch isoliert. Ein weiterer auf dem Träger 18 angeordneter metallischer Abschnitt 22 kann die beide Abschnitte 19, 20 umgeben und so für ein einheitliches elektrisches Potenzial entlang des Rands des Trägers 18 sorgen.

Die Abschnitte 19, 20 sind über verschiedene Adern der Leitung 14 mit dem Gerät 11 verbunden. Optional kann (alternativ oder zusätzlich) der Abschnitt 22 über eine Ader der Leitung 14 mit dem Gerät 11 verbunden sein.

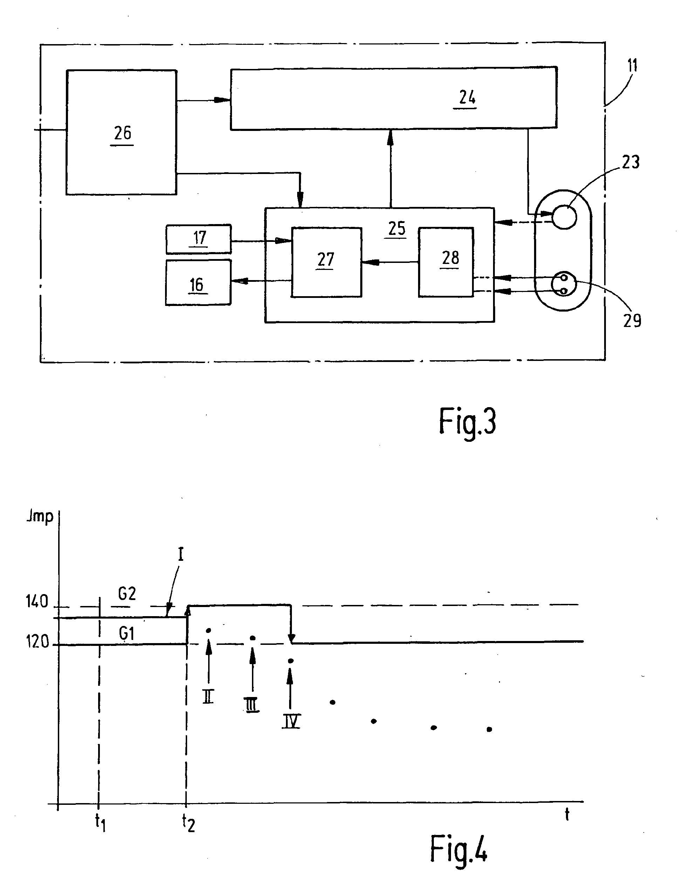

Figur 3 veranschaulicht den prinzipiellen Aufbau des Geräts 11. Eine zum Anschluss der Leitung 12 und somit des Instruments 13 vorgesehene Buchse 23 ist mit einem elektrischen Generator 24, bspw. einem HF-Generator verbunden. Die Buchse 23 kann ein oder mehrpolig ausgeführt sein. Zusätzlich zu einem die HF-Leistung des Generators 24 übermittelnden Kontakt kann sie einen oder mehrere Kontakte für weitere Adern der Leitung 12 aufweisen, über die zum Beispiel Steuersignale an eine Steuereinrichtung 25 des Geräts 11 gegeben werden. Die entsprechende Leitungsverbindung ist in Figur 3 gestrichelt eingetragen.

Die zentrale Steuereinrichtung 25 steuert den Generator 24. Dieser wird wie auch die Steuereinrichtung 25 von einem Netzteil 26 mit Betriebsspannung versorgt. Die Steuereinrichtung 25 ist mit den Bedienelementen 17 und der Anzeigeeinrichtung 16 verbunden. Sie weist ein zentrales Verarbeitungsmodul 27 auf, das einen oder mehrere Rechner, zum Beispiel Mikrorechner umfassen kann, um die nachfolgend beschriebenen Funktionen zu erbringen.

Außerdem umfasst die Steuereinrichtung 25 ein Modul 28 zur Bestimmung der Impedanz zwischen den Abschnitten 19, 20 der Neutralelektrode 15, wenn diese an einem Patienten befestigt ist. Dazu ist das Modul 28 mit zwei Kontakten eines Anschlusses beispielsweise in Form einer Buchse 29 verbunden, an die die Leitung 14 anzuschließen ist. Die beiden Kontakte sind verschiedene Adern der Leitung 14 zugeordnet, von denen eine zu dem Abschnitt 19 und die andere zu dem Abschnitt 20 der Neutralelektrode 15 führt. Die beiden Kontakte sind wie aus Figur 3 ersichtlich mit dem Modul 28 verbunden.

Das Modul 28 erfasst die Impedanz oder den Scheinwiderstand zwischen den beiden Abschnitten 19, 20 zum Beispiel kontinuierlich oder intermittierend. Es kann dazu fortwährend oder wiederholt eine geringe Spannung an die Abschnitte 19, 20 anlegen und den sich einstellenden Strom erfassen. Alternativ kann ein vorgegebener Strom angelegt und die Spannung erfasst werden. Der jeweils zum Messen verwendete Strom und/oder die Spannung können Gleichspannung, Wechselspannung bzw. Gleichstrom oder Wechselstrom geeigneter Kurvenform und Frequenz sein. Vorzugsweise wird die Impedanz oder der Scheinwiderstand zwischen den Abschnitten 19, 20 ausgewertet. Es kann jedoch auch vorgesehen sein, ausschließlich den Realteil oder ausschließlich den Imaginärteil der Impedanz zu erfassen und diesen in dem nachfolgend beschriebenen Verfahren als Kennzeichen für die Anlagequalität der Neutralelektrode zu nutzen.

Das Modul 28 kann auch darauf ausgelegt sein, die Impedanz zwischen einem der Abschnitte 19, 20 und dem Abschnitt 22 zu bestimmen. Die Neutralelektrode kann auch in mehrere Abschnitte aufgeteilt sein, zwischen denen das Modul 28 die Impedanz bestimmt. In jeder Ausführungsform kann die Impedanzmessung dauernd oder intermittierend erfolgen. Bei Impedanzmessung zwischen mehreren Leiterflächenpaaren kann diese gleichzeitig oder seriell erfolgen. Auf jedes Leiterflächenpaar kann das hier beschriebene Verfahren angewendet werden.

Das Modul 28 kann Teil der Steuereinrichtung oder als gesonderte Baugruppe aufgebaut sein. Jedenfalls aber liefert es kontinuierlich oder wiederholt oder auch lediglich einmalig ein Signal an das Verarbeitungsmodul 27, wobei dieses Signal die erfasste Impedanz oder daraus abgeleitete Größen kennzeichnet. Das Verarbeitungsmodul 27 bestimmt dann, ob die erfasste Impedanz unter einem ersten Grenzwert G1 sowie über oder unter einem zweiten Grenzwert G2 liegt. Der erste Grenzwert G1 beträgt beispielsweise 120 Ohm. Der zweite Grenzwert G2 beträgt beispielsweise 140 Ohm. Die Grenzwert G1 und G2 können jedoch abweichend von diesen Zahlenwerten festgelegt sein.

In Figur 4 ist die Funktionsweise des Verarbeitungsmoduls 27 anhand eines Diagramms veranschaulicht. Liegt die von dem Modul 28 bestimmte Impedanz Imp unter dem Grenzwert G1, gibt die Steuereinrichtung 25 den Betrieb des Generators 24 uneingeschränkt frei. Es wird von einer korrekt angelegten Neutralelektrode 15 ausgegangen. Zum Beispiel können nun über die Buchse 23 empfangene Anforderungssignale, die Steuereinrichtung 25 veranlassen, den Generator 24 zu aktivieren, so dass an dem Instrument 13 HF-Leistung zur Verfügung steht. Ein Chirurg kann dann ohne Weiteres die gewünschten operativen Eingriffe vornehmen.

Figur 4 veranschaulicht einen Zustand, bei dem die Impedanz Imp gemäß der Kurve I oberhalb des ersten Grenzwerts G1, jedoch unterhalb des zweiten Grenzwerts G2 liegt. Dies kann verschiedene Gründe haben. Zum Beispiel ist es möglich, dass die Neutralelektrode 15 nicht korrekt angelegt ist. Es ist auch möglich, dass die Impedanz Imp aufgrund trockener Haut oder anderer Spezifika des Patienten erhöht ist. Das Gerät 11 beginnt zu einem Zeitpunkt t1 eine Impedanzmessung und stellt fest, dass die Impedanz Imp zwischen den Grenzwerten G1 und G2 liegt. Sie sperrt zunächst den normalen Betrieb, so dass der Generator 24 nicht mehr betrieben werden kann. Über die Anzeigeeinrichtung 16 wird ein Signal ausgegeben, dass den Bediener auffordert das korrekte Anliegen der Neutralelektrode 15 zu überprüfen.

Ist dies geschehen, kann der Bediener z.B. zu einem Zeitpunkt t2 über eines der Bedienelemente 17 die korrekte Anlage der Elektrode bestätigen und somit den Betrieb des Generators freigeben. Es gilt nun der zweite Grenzwert G2 als obere Grenze für die Impedanz Imp. Sofern und solange diese nicht überschritten wird, ist der Betrieb freigegeben. Würde sie jedoch überschritten, wäre der Betrieb des Generators 24 wiederum (nichtüberwindbar) gesperrt.

In dem Beispiel nach Figur 4 ist der Betrieb des Geräts 11 nun möglich, weil die Impedanz Imp zwischen G1 und G2 liegt, die korrekte Anlage der Neutralelektrode am Patienten überprüft und bestätigt wurde. Es wird nun angenommen, dass der Chirurg das Instrument 13 betätigt. Deswegen oder auch unabhängig davon kann sich die Impedanz Imp z.B. durch Anfeuchtung der Haut unter der Neutralelektrode 15 mit der Zeit verringern. Optional kann vorgesehen sein, dass die Steuereinrichtung 25 die Impedanz fortwährend oder wie dargestellt zu diskreten Zeitpunkten ermittelt. Während entsprechende Messwerte II und III noch über dem ersten Grenzwert G1 liegen, liegt ein weiterer Messwert IV schon unterhalb des ersten Grenzwerts G1. Dies kann auf Schweißbildung unter der Neutralelektrode 15 zurückgehen. Bei einer bevorzugten Ausführungsform schaltet nun das Verarbeitungsmodul 27 wieder den ersten Grenzwert G1 als Grenzwert für den Betrieb des Geräts 11 aktiv. Sollte er wieder überschritten werden, wird das Gerät wieder, wie eingangs schon beschrieben, zumindest vorläufig gesperrt.

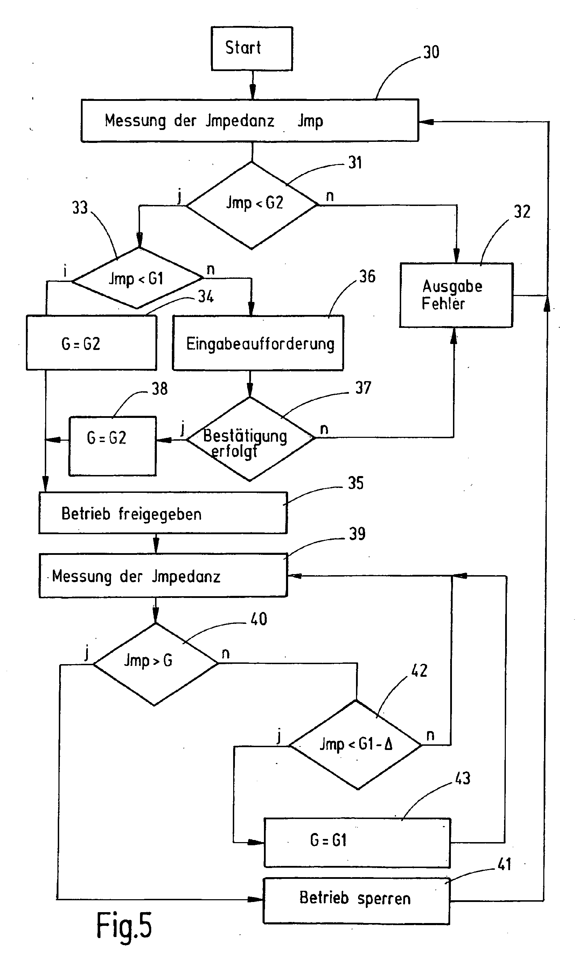

Das erfindungsgemäße Verfahren kann nach dem aus Figur 5 ersichtlichen Flussbild ausgeführt werden. Nach dem Beginn der Abarbeitungsprozedur wird in einem Block 30 die Impedanz Imp zwischen den Abschnitten 19 und 20 gemessen. Zunächst wird dann in einen Block 31 die Impedanz Imp mit dem zweiten Grenzwert G2 von z.B. 140 Ohm verglichen. Übersteigt die Impedanz den zweiten Grenzwert G2 geht das System zu einem Block 32 über, in dem der Betrieb des Generators 24 unterbunden und auf dem Bildschirm der Anzeigeeinrichtung 16 eine Fehlermeldung ausgegeben wird. Der Prozess wiederholt von dort ausgehend die Messung der Impedanz Imp.

Ist die Impedanz Imp im Block 31 hingegen kleiner als der zweite Grenzwert G2 (oder gleich) wird nachfolgend in Block 33 geprüft, ob die Impedanz Imp kleiner ist als der erste Grenzwert G1 (oder gleich). Ist dies der Fall, wird in Block 34 eine Grenzwertvariable G = G1 gesetzt und in Block 35 der Betrieb des Generators 24 freigegeben. Wird jedoch festgestellt, dass die Impedanz Imp größer als G1 ist, d.h. zwischen G1 und G2 liegt, wird zu Block 36 verzweigt. In diesem wird der Nutzer zur Bestätigung aufgefordert, dass die Neutralelektrode 15 korrekt anliegt. Bestätigt der Nutzer die korrekte Anlage der Neutralelektrode in Block 37, wird in Block 38 die Grenzwertvariable G = G2 gesetzt und der Betrieb in Block 35 freigeben. Erfolgt die Bestätigung nicht oder nicht innerhalb einer vergebenen Zeitspanne, wird zu Block 32 oder alternativ zu Block 30 verzweigt. Das heißt es wird mit oder ohne Ausgabe einer Fehlermeldung die Impedanz erneut gemessen.

Wenn der Betrieb freigegeben worden ist, wird nach Abarbeitung des Blocks 35 die Impedanz in Block 39 wiederum gemessen. Nachfolgend wird in Block 40 geprüft, ob die gemessen Impedanz größer als die Grenzwertvariable G ist. Ist dies der Fall, wird der Betrieb gesperrt und zu Block 41, zu Block 32 oder 30 zurückgekehrt. Ist dies jedoch nicht der Fall, wird zu Block 42 verzweigt. In diesem wird geprüft, ob die Impedanz kleiner als der erste Grenzwert G1 abzüglich eines Hysteresewerts Δ ist. Ist dies nicht der Fall, wird zu Block 39 verzweigt. Ist die Impedanz Imp hingegen kleiner als der erste Grenzwert G1-Δ wird in Block 43 der Grenzwert G wieder auf den ersten Grenzwert G1 zurückgesetzt und zu Block 39 rückverzweigt.

Das vorstehend erläuterte Flussbild zeigt die Nutzung unterschiedlicher Grenzwerte G1 und G2. Wird der Grenzwert G1 für die Impedanz überschritten, kann der Nutzer durch entsprechende Eingabe willkürlich die Sicherheitsabschaltschwelle des Geräts 11 auf den zweiten Grenzwert G2 heraufsetzen. Eine Überschreitung dieses Grenzwerts G2 ist jedoch aus Sicherheitsgründen nicht möglich.

Mit dem erfindungsgemäßen Verfahren wird es dem Bediener eines elektrochirurgischen Geräts 11 ermöglicht, dessen Sicherheitsabschaltschwelle für den Übergangswiderstand an der Neutralelektrode 15 von einem ersten niedrigen Wert G1 auf einen zweiten etwas höheren Wert G2 zu verschieben, um bei relativ hochohmigen Patienten, bei denen trotz korrekt anliegender Neutralelektrode 15 der Übergangswiderstand etwas erhöht ist, einen ungestörten Betrieb des Geräts 11 und des Instruments 13 zu ermöglichen.

### Bezugszeichenliste:

- 10: elektrochirurgische Einrichtung
- 11: Elektrochirurgisches Gerät
- 12: Leitung
- 13: chirurgisches Instrument
- 14: Leitung
- 15: Neutralelektrode
- 16: Anzeigeeinrichtung
- 17: Bedienelemente
- 18: Träger
- 19, 20: metallische Abschnitte der Neutralelektrode
- 21: Abstand
- 22: Abschnitt
- 23: Buchse
- 24: Generator
- 25: Steuereinrichtung
- 26: Netzteil
- 27: Verarbeitungsmodul
- 28: Modul
- 29: Buchse, Anschluss
- I: Kurve
- II, III, IV: Messwerte
- 30 - 43: Blöcke

## Patentansprüche

1. Verfahren zur Steuerung eines elektromedizinischen Geräts (11) in Abhängigkeit von der an der Neutralelektrode (15) erfassten Impedanz, wobei bei dem Verfahren:
der Betrieb des elektromedizinischen Geräts (11) gesperrt wird, wenn
die elektrische Impedanz (Imp) zwischen wenigstens zwei Abschnitten (19, 20) der an einem Patienten befestigten Neutralelektrode (15) einen ersten Grenzwert (G1) überschreitet,
wobei ein Warnsignal erzeugt und bei Eingabe eines entsprechenden Nutzerbefehls die Sperrung aufgehoben wird;
**dadurch gekennzeichnet, dass** die Aufhebung der Sperrung nur dann erlaubt ist, wenn die elektrische Impedanz (Imp) einen zweiten Grenzwert (G2) unterschreitet, der größer ist als der erste Grenzwert (G1).

2. Verfahren nach Anspruch 1, **dadurch gekennzeichnet, dass** die Aufhebung der Sperrung nur vorgenommen wird, wenn die Impedanz einen zweiten Grenzwert (G2) unterschreitet, der höher ist als der erste Grenzwert (G1).

3. Verfahren nach Anspruch 1, **dadurch gekennzeichnet, dass** der Betrieb des elektromedizinischen Geräts (11) nichtaufhebbar zumindest so lange gesperrt wird, wie die Impedanz (Imp) einen zweiten Grenzwert (G2) überschreitet, der höher ist als der erste Grenzwert (G1).

4. Verfahren nach Anspruch 1 oder 3, **dadurch gekennzeichnet, dass** die Impedanz (Imp) während des Betriebs des elektromedizinischen Geräts (11) wiederholt oder fortwährend überwacht wird.

5. Verfahren nach Anspruch 1, **dadurch gekennzeichnet, dass** bei Überschreitung des ersten Grenzwerts (G1) und nachfolgender manueller Aufhebung der Sperrung des Betriebs die Impedanz (Imp) fortwährend oder wiederholt überwacht wird wobei, wenn ein Unterschreiten des ersten Grenzwerts (G1) festgestellt wird, nachfolgend überwacht wird, ob der erste Grenzwert (G1) wieder überschritten wird, um in einem solchen Fall eine Warnung auszugeben und/oder den Betrieb des elektromedizinischen Geräts (11) erneut zu sperren.

6. Verfahren nach Anspruch 5, **dadurch gekennzeichnet, dass** im Falle der erneuten Sperrung wiederum die Eingabe eines Eingabesignals erwartet wird, um die Sperrung aufzuheben.

7. Verfahren nach Anspruch 1, **dadurch gekennzeichnet, dass** im Falle einer Sperrung zur Überprüfung der korrekten Anlage der Neutralelektrode (15) aufgefordert wird.

8. Verfahren nach Anspruch 1, **dadurch gekennzeichnet, dass** im Falle einer erneuten Sperrung zur Anlage einer größeren Neutralelektrode (15) aufgefordert wird.

9. Verfahren nach Anspruch 1, **dadurch gekennzeichnet, dass** das elektromedizinische Gerät (11) in gesperrtem Zustand bei Überschreitung des ersten Grenzwerts (G1) und Unterschreitung des zweiten Grenzwerts (G2) mit reduzierter Leistung betreibbar ist.

10. Elektrochirurgisches Gerät (11)
mit einem elektrischen Generator (24) zur Speisung eines elektrochirurgischen Instruments (13) zur Bewirkung chirurgischer Effekte an biologischem Gewebe eines Patienten,
mit einem Anschluss (29) für eine Neutralelektrode (15) zur Anlage an den Patienten, die zumindest zwei voneinander elektrisch isolierte Abschnitte (19, 20) aufweist,
mit einer Steuereinrichtung (25), die ein Modul (28) zur Erfassung der elektrischen Impedanz (Imp) zwischen den beiden Abschnitten (19, 20) aufweist und die mit dem elektrischen Generator (24) verbunden ist,
wobei die Steuereinrichtung ausgelegt ist, zu bestimmen ob die erfasste Impedanz (Imp) einen ersten Grenzwert (G1) überschreitet, bei Überschreitung des ersten Grenzwerts (G1) den Betrieb des Generators zu sperren, ein Warnsignal auszugeben, sowie bei Eingabe eines entsprechenden Nutzerbefehls die Sperrung aufzuheben,
**dadurch gekennzeichnet, dass** die Steuereinrichtung weiterhin ausgelegt ist, zu bestimmen ob die erfasste Impedanz (Imp) einen zweiten Grenzwert (G2) unterschreitet, der größer ist als der erste Grenzwert (G1), und nur bei Unterschreitung des zweiten Grenzwerts (G2) die Aufhebung der Sperrung zu erlauben.

11. Gerät nach Anspruch 10, **dadurch gekennzeichnet, dass** der Generator (24) ein HF-Generator ist.

12. Gerät nach Anspruch 10, **dadurch gekennzeichnet, dass** die Steuereinrichtung (25) den Betrieb des Generators (24) nichtaufhebbar sperrt, wenn die Impedanz (Imp) den zweiten Grenzwert (G2) überschreitet.

13. Gerät nach Anspruch 10, **dadurch gekennzeichnet, dass** die Steuereinrichtung (25) die Impedanz (Imp) während des Betriebs des Generators (24) wiederholt oder fortwährend überwacht.

14. Gerät nach Anspruch 10, **dadurch gekennzeichnet, dass** die Steuereinrichtung (25) bei Überschreitung des ersten Grenzwerts (G1) und nachfolgender manueller Aufhebung der Sperrung des Betriebs des Generators (24) die Impedanz (Imp) fortwährend oder wiederholt überwacht, wobei sie, wenn ein Unterschreiten des ersten Grenzwerts (G1) festgestellt wird, nachfolgend überwacht, ob der erste Grenzwert (G1) wieder überschritten wird, um in einem solchen Fall eine Warnung auszugeben und/oder den Betrieb des Generators (24) erneut zu sperren.

15. Gerät nach Anspruch 14, **dadurch gekennzeichnet, dass** die Steuereinrichtung (25) im Falle der erneuten Sperrung wiederum die Eingabe eines Eingabesignals erwartet, um die Sperrung aufzuheben.

## Claims

1. Process for controlling an electromedical device (11) in dependence on the impedance detected at the neutral electrode (15), wherein during the process:
the operation of the electromedical device (11) is disabled when the electrical impedance (Imp) between at least two sections (19, 20) of the neutral electrode (15) attached to a patient exceeds a first limit value (G1), wherein a warning signal is generated and the disabling action is cancelled on input of a corresponding user command,
**characterised in that** the cancellation of the disabling action is only permitted when the electrical impedance (Imp) is below a second limit value (G2), which is higher than the first limit value (G1).

2. Process according to claim 1, **characterised in that** the cancellation of the disabling action is only performed when the impedance is below a second limit value (G2), which is higher than the first limit value (G1).

3. Process according to claim 1, **characterised in that** the operation of the electromedical device (11) is disabled without the ability to be cancelled at least until the impedance (Imp) exceeds a second limit value (G2), which is higher than the first limit value (G1).

4. Process according to claim 1 or 3, **characterised in that** the impedance (Imp) is monitored repeatedly or continuously during the operation of the electromedical device (11 ).

5. Process according to claim 1, **characterised in that** when the first limit value (G1) has been exceeded and the disabling of the operation has subsequently been manually cancelled, the impedance (Imp) is monitored continuously or repeatedly and, if it is determined that it has fallen below the first limit value (G1), it is subsequently monitored to determine whether the first limit value (G1) is exceeded again in order to emit a warning in such a case and/or disable the operation of the electromedical device (11) once again.

6. Process according to claim 5, **characterised in that** in the event of renewed disabling action the input of an input signal is awaited again to cancel the disabling action.

7. Process according to claim 1, **characterised in that** in the event of a disablement a command to monitor the correct placement of the neutral electrode (15) is made.

8. Process according to claim 1, **characterised in that** in the event of a renewed disablement a command for placement of a larger neutral electrode (15) is made.

9. Process according to claim 1, **characterised in that** the electromedical device (11) is operable in the disabled state at reduced power when the first limit value (G1) is exceeded and the impedance is below the second limit value (G2).

10. Electrosurgical device (11),
with an electric generator (24) for supplying an electrosurgical instrument (13) to perform surgical actions on biological tissue of a patient,
with a connection (29) for a neutral electrode (15) for placement on a patient, which has at least two sections (19, 20) electrically insulated from one another,
with a control device (25), which has a module (28) for detecting the electrical impedance (Imp) between the two sections (19, 20) and which is connected to the electric generator (24),
wherein the control device is designed to determine whether the detected impedance (Imp) exceeds a first limit value (G1), to disable the operation of the generator when the first limit value (G1) is exceeded, to emit a warning signal and also to cancel the disabling action on input of a corresponding user command,
**characterised in that** the control device is additionally designed to determine whether the detected impedance (Imp) is below a second limit value (G2), which is higher than the first limit value (G1) and to only allow the cancellation of the disabling action when the impedance is below the second limit value (G2).

11. Device according to claim 10, **characterised in that** the generator (24) is a HF generator.

12. Device according to claim 10, **characterised in that** the control device (25) is disabled without the ability to be cancelled when the impedance (Imp) exceeds the second limit value (G2).

13. Device according to claim 10, **characterised in that** the control device (25) monitors the impedance (Imp) repeatedly or continuously during the operation of the generator (24).

14. Device according to claim 10, **characterised in that** when the first limit value (G1) has been exceeded and the disabling of the operation has subsequently been manually cancelled, the control device (25) monitors the impedance (Imp) continuously or repeatedly and, if it is determined that it has fallen below the first limit value (G1), it subsequently monitors whether the first limit value (G1) is exceeded again in order to emit a warning in such a case and/or disable the operation of the generator (24) once again.

15. Device according to claim 14, **characterised in that** in the event of renewed disabling action the control device (25) awaits the input of an input signal again to cancel the disabling action.

## Revendications

1. Procédé de commande d'un appareil électromédical (11) en fonction de l'impédance enregistrée sur l'électrode neutre (15), sachant qu'avec le procédé :
le fonctionnement de l'appareil électromédical (11) est bloqué, si l'impédance électrique (Imp) entre au moins deux parties (19, 20) de l'électrode neutre (15) fixée sur un patient dépasse une première valeur limite (G1), un signal d'avertissement étant produit et le blocage étant supprimé en cas d'introduction d'une commande d'utilisateur correspondante,
**caractérisé en ce que** la suppression du blocage n'est autorisée que si l'impédance électrique (Imp) est inférieure à une deuxième valeur limite (G2) qui est supérieure à la première valeur limite (G1).

2. Procédé selon la revendication 1, **caractérisé en ce que** la suppression du blocage n'est effectuée que si l'impédance est inférieure à une deuxième valeur limite (G2) qui est supérieure à la première valeur limite (G1).

3. Procédé selon la revendication 1, **caractérisé en ce que** le fonctionnement de l'appareil électromédical (11) est bloqué sans possibilité de suppression, au moins tant que l'impédance (Imp) dépasse une deuxième valeur limite (G2) qui est supérieure à la première valeur limite (G1).

4. Procédé selon la revendication 1 ou 3, **caractérisé en ce que** l'impédance (Imp) est surveillée plusieurs fois ou en continu au cours du fonctionnement de l'appareil électromédical (11).

5. Procédé selon la revendication 1, **caractérisé en ce qu'**en cas de dépassement de la première valeur limite (G1) et suppression manuelle subséquente du blocage du fonctionnement, l'impédance (Imp) est surveillée plusieurs fois ou en continu, sachant que si un dépassement de la première valeur limite (G1) vers le bas est constaté, on surveille ensuite si la première valeur limite (G1) est de nouveau dépassée vers le haut, pour émettre dans ce cas un avertissement et/ou bloquer de nouveau le fonctionnement de l'appareil électromédical (11).

6. Procédé selon la revendication 5, **caractérisé en ce qu'**en cas de nouveau blocage, l'introduction d'un signal d'entrée est de nouveau attendue pour supprimer le blocage.

7. Procédé selon la revendication 1, **caractérisé en ce qu'**en cas de blocage, une demande est émise pour vérifier l'application correcte de l'électrode neutre (15).

8. Procédé selon la revendication 1, **caractérisé en ce qu'**en cas de nouveau blocage, une demande est émise pour appliquer une électrode neutre (15) plus grande.

9. Procédé selon la revendication 1, **caractérisé en ce qu'**à l'état bloqué, en cas de dépassement de la première valeur limite (G1) vers le haut et de dépassement de la deuxième valeur limite (G2) vers le bas, l'appareil électromédical (11) peut être exploité avec une puissance réduite.

10. Appareil électromédical (11),
comprenant un générateur électrique (24) pour alimenter un instrument électrochirurgical (13), afin d'obtenir des effets chirurgicaux sur des tissus biologiques d'un patient,
comprenant une connexion (29) pour une électrode neutre (15) pour l'application sur le patient, qui présente au moins deux parties (19, 20) isolées électriquement l'une de l'autre,
comprenant un dispositif de commande (25) qui présente un module (28) pour enregistrer l'impédance électrique (Imp) entre les deux parties (19, 20) et qui est relié au générateur électrique (24), le dispositif de commande étant conçu pour déterminer si l'impédance (Imp) enregistrée dépasse une première valeur limite (G1) et, si la première valeur limite (G1) est dépassée, pour bloquer le fonctionnement du générateur, émettre un signal d'avertissement et supprimer le blocage en cas d'introduction d'une commande d'utilisateur correspondante,
**caractérisé en ce que** le dispositif de commande est en outre conçu pour déterminer si l'impédance (Imp) enregistrée est inférieure à une deuxième valeur limite (G2) qui est supérieure à la première valeur limite (G1), et pour autoriser la suppression du blocage, uniquement en cas de dépassement en dessous de la deuxième valeur limite (G2).

11. Appareil selon la revendication 10, **caractérisé en ce que** le générateur (24) est un générateur HF.

12. Appareil selon la revendication: 10, **caractérisé en ce que** le dispositif de commande (25) bloque le fonctionnement du générateur (24) sans possibilité de suppression, si l'impédance (Imp) dépasse la deuxième valeur limite (G2).

13. Appareil selon la revendication 10, **caractérisé en ce que** le dispositif de commande (25) surveille l'impédance (Im) plusieurs fois ou en continu pendant le fonctionnement du générateur (24).

14. Appareil selon la revendication 10, **caractérisé en ce qu'**en cas de dépassement de la première valeur limite (G1) et suppression manuelle subséquente du blocage du fonctionnement du générateur (24), le dispositif de commande (25) surveille l'impédance (Imp) plusieurs fois ou en continu, sachant que si un dépassement de la première valeur limite (G1) vers le bas est constaté, il surveille ensuite si la première valeur limite (G1) est de nouveau dépassée vers le haut, pour émettre dans ce cas un avertissement et/ou bloquer de nouveau le fonctionnement du générateur (24).

15. Appareil selon la revendication 14, **caractérisé en ce qu'**en cas de nonveau blocage, le dispositif de commande (25) attend de nouveau l'introduction d'un signal d'entrée pour supprimer le blocage.
